# EUROPEAN PATENT SPECIFICATION

(11) **EP 1 493 020 B1**
(45) Date of publication and mention of the grant of the patent: **07.06.2006**
(21) Application number: 02730145.6
(22) Date of filing: 11.04.2002
(51) Int. Cl.: G01N 21/72, A61F 9/02

(54) **METHOD AND DEVICE FOR VIEWING A BURNER FLAME**
VORRICHTUNG UND VERFAHREN ZUR BEOBACHTUNG DER FLAMME EINES BRENNERS
PROCEDE ET DISPOSITIF PERMETTANT DE VISUALISER UNE FLAMME DE BRULEUR

(43) Date of publication of application: 05.01.2005
(73) Proprietor: Borealis Technology Oy, 06101 Porvoo (FI)
(72) Inventor: EDWIN, Emil, N-7043 Trondheim (NO); ARNESEN, Tore, N-7053 Ranheim (NO)
(74) Representative: Power, Philippa Louise
(86) International application number: PCT/EP2002/004044
(87) International publication number: WO 2003/085388

(56) References cited:
- WO-A-99/50649
- GB-A- 1 128 625
- GB-A- 1 605 192
- US-A- 4 466 943
- US-A- 4 820 046

## Description

The present invention relates to a method and apparatus for viewing a flame in the pyrolysis section of a petrochemical cracker reactor.

In a petroleum cracker, large hydrocarbon molecules such as ethane and propane from natural gas, or heavier liquids such as naphta and gas oil from petroleum are split into smaller molecules. This is often done to provide olefins such as ethylene that are useful in themselves, or may be used in polymerisation processes.

In the case of ethane and propane, the gas is heated to above about 800°C at which point bonds within the molecule break, producing a range of smaller molecules. The desired products are then separated out. The same principle applies when cracking heavier substances, but since the molecules are much larger, a far greater range of smaller molecules is provided. Although some processes provide a smaller yield of olefins, many other useful by-products are produced.

In a typical ethane cracker plant, the cracking takes place in a pyrolysis section. Here, ethane is pumped through a maze of 100-150mm diameter tubes located within a furnace where it is heated up to about 800°C and cracks. The ethane never comes into direct contact with the source of heat, if it were to do so it would ignite disastrously.

Typically, the pyrolysis section includes a plurality of burners which are positioned adjacent the tubes through which the ethane is pumped. The burners combust a fuel such as natural gas in order to heat the gas in the tubes to the required temperature.

To achieve maximum thermal efficiency of the cracker the flame from each of the burners should be maintained at the required size and orientation. This can be done by adjusting the quantity of fuel supplied to the burner and/or by adjusting the ratio of fuel supplied to air and/or by adjusting the direction of the jets of fuel.

In order to monitor the size and orientation of the burner flames, an operator views the flames at regular intervals and then makes any adjustments which may be required. However, the heat in the pyrolysis section is such that the walls of the cracker are heated to a temperature of about 1100°C. Consequently, radiant heat is given off from the walls to create a bright background against which it is very difficult to see the burner flames.

In the past, this problem has been overcome by adding either copper or sodium bicarbonate to the flames to provide a colour which is visible against the bright background of the walls of the cracker. To do this however, the pyrolysis chamber must be opened up and the copper or sodium bicarbonate thrown into the flames. It will be appreciated that at the operating temperatures in question this is a complex procedure which results in significant inconvenience and energy losses.

The present invention seeks to provide a method of viewing the flames of the burners in a furnace such as the pyrolysis chamber of a cracker which can be carried out quickly and easily and without the need to lose heat from the furnace.

The applicants have realised that if the light emitted by the burner flames or a part of that light could be separated from the background radiant light in the furnace, the burner flames could then be viewed without the need for complex solutions such as throwing additives into the flames.

US-A-4,820,046 discloses a method of viewing the flame produced by a burner in a furnace, comprising viewing the flame through an interference filter.

From a first aspect the present invention is characterised over US-A-4,820,046 in that the furnace is the pyrolysis section of a petroleum cracker; the fuel burnt by the burner is natural gas; and the interference filter is adapted to pass light of the wavelength of sodium only.

Thus, the interference filter acts to block out the bulk of the ambient light of the furnace such that the burner flame is clearly visible.

In the method of the invention, the furnace is the pyrolysis section of a petroleum cracker. The method is particularly advantageous in such an environment as the walls of the cracker are heated to a very high temperature such that they emit significant levels of white light which makes it difficult or even impossible to see the flame of a burner in the cracker under normal circumstances.

Typically, the fuel which is burnt in the petroleum cracker is natural gas and most typically, a mixture of hydrogen, methane and air. Tests have shown that this fuel can contain traces of sodium. The reason for this is not known but it is thought to be because methane and natural gas often come from environments in which salt is present. In the method of the invention therefore, the filter used is a sodium interference filter which filters out substantially all the light other than the sodium light emitted by the sodium trace elements in the fuel.

Preferably the sodium interference filter has a pass bandwidth of approximately 10nm so that light of wavelength 0.584 to 0.594µm may pass through the filter. More preferably the pass bandwidth is 2 to 5nm and, still more preferably, the pass bandwidth is 1nm so that only light of wavelength 0.589µm passes through the filter.

The use of a sodium interference filter is particularly advantageous as there is effectively no light of the wavelength of sodium present as ambient light in the furnace such that the burner flame is very clearly visible using this method.

The use of a sodium interference filter to view a burner flame in a furnace is considered to be novel and inventive in its own right and so, from a further aspect, the present invention provides a method according to claim 1 viewing the flame produced by a burner in a furnace, comprising viewing the flame through an interference filter adapted to pass light of the wavelength of sodium only.

The sodium interference filter could take any form and the burner in the furnace could be viewed through a door which is opened in use as in known systems. Preferably however, a window is provided in the wall of the furnace through which the burner can be viewed. This has the advantage that the furnace does not need to be opened each time that the burners are viewed. Thus the temperature inside the furnace is not disturbed and thermal currents which can distort the action of the burner flame are not created by opening and closing the furnace at regular intervals.

Preferably, the window is made of quartz which is a material capable of withstanding the temperature gradient across the wall of the furnace while also providing the necessary transparency.

In one preferred embodiment, the interference filter could be provided as a panel attached to the window of the furnace.

Still more preferably, the filter is a panel which can be placed over the window or removed by a user as required. Thus for example, the filter could be hinged to the wall of the furnace to allow quick and easy adjustment thereof.

In an alternative embodiment, a pair of glasses or goggles comprising an interference filter in each lens thereof is provided. This has the advantage of allowing a user to carry the glasses with him for example from one furnace to the next. The goggles have the additional advantage that they could also be used with a traditional furnace in which no sealed window is provided but a door is merely opened when a user wishes to look inside the furnace.

In a still further preferred embodiment of the invention, the interference filter could be provided in a camera arranged inside the furnace and adapted to photograph the burner at regular intervals. The information from the camera could then be relayed to an operator who could make any necessary adjustments to the burner from a remote location. This would clearly be advantageous in a large scale refinery or similar scale production plant where considerable numbers of personnel would be required to monitor the operation of each furnace in situ.

Ideally the camera could be programmed to photograph the burner about once every 10 minutes.

It will be appreciated that the furnace would normally include a plurality of burners and, in the case of a petroleum cracker, ten or more burners could be provided. Thus if necessary, the camera could be programmed to move along a row of burners and to take several pictures of respective burners or groups thereof.

From a further aspect, the invention provides an apparatus comprising a furnace, a burner in the furnace and an apparatus for viewing the flame produced by the burner, the apparatus for viewing the flame comprising an interference filter, wherein the furnace is the pyrolysis section of a petroleum cracker, the burner is for burning natural gas, and the interference filter is adapted to pass light of the wavelength of sodium only.

In one preferred embodiment, the apparatus further comprises a window provided in the wall of the furnace through which the burner can be viewed.

Preferably, the window is made of quartz.

In one preferred embodiment, the interference filter could be provided as a panel attached to the sealed window of the furnace.

Still more preferably, the filter is a panel which can be placed over the window or removed by a user as required. Thus for example, the filter could be hinged to the wall of the furnace to allow quick and easy adjustment thereof.

In an alternative embodiment, the apparatus comprises a pair of glasses or goggles comprising an interference filter in each lens thereof.

In another alternative embodiment of the invention, the apparatus comprises a camera in which the interference filter is provided, wherein the camera is arranged inside the furnace and adapted to photograph the burner at regular intervals. The information from the camera could then be relayed to an operator who could make any necessary adjustments to the burner from a remote location.

Ideally the camera could be programmed to photograph the burner about once every 10 minutes.

It will be appreciated that the furnace would normally include a plurality of burners and, in the case of a petroleum cracker, ten or more burners could be provided. Thus if necessary, the camera could be programmed to move along a row of burners and to take pictures of respective burners or groups thereof.

Preferred embodiments of the invention will now be described, by way of example only, and with reference to the accompanying drawings in which:
Figure 1 schematically shows a pyrolysis section of a petroleum cracker; and
Figure 2 shows a pair of goggles according to one embodiment of the invention.

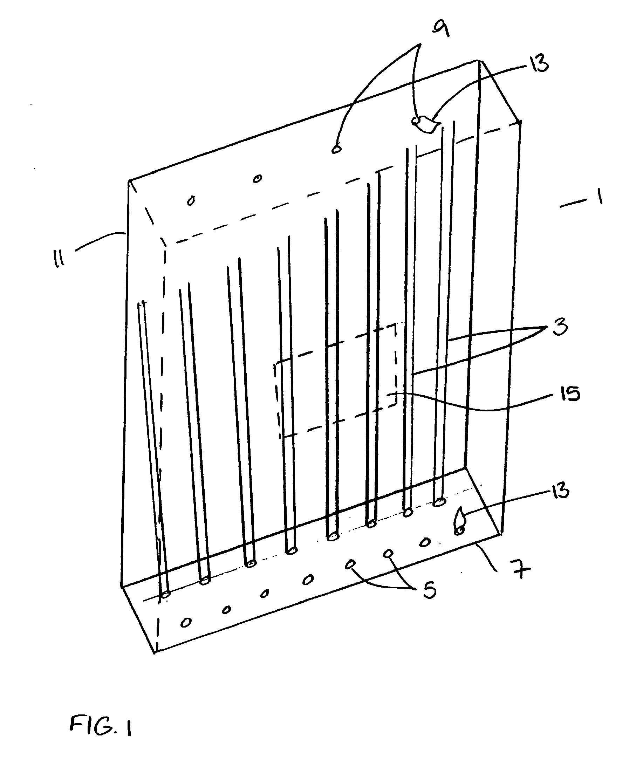

As shown in Figure 1, a petroleum cracker includes a pyrolysis section 1 in which petroleum is heated in order to carry out the cracking process. The pyrolysis section includes a number of pipes or tubes 3 through which the petroleum flows in use. Burners 5 are provided in the base 7 of the pyrolysis section and further burners 9 are also provided in the rear wall 11. The burners 5, 9 burn a mixture of hydrogen, air and methane supplied to them by a pipe network (not shown).

The burners each produce a naked flame 13, the size and orientation of which must be controlled in order to ensure even heating of the petroleum pipes 3. In order to monitor the size and orientation of the flames 13, a window 15 made of quartz is provided in a wall of the pyrolysis section. The window is sealed so that the temperature and stability of the burner flames inside the pyrolysis section are not affected by air currents from the window.

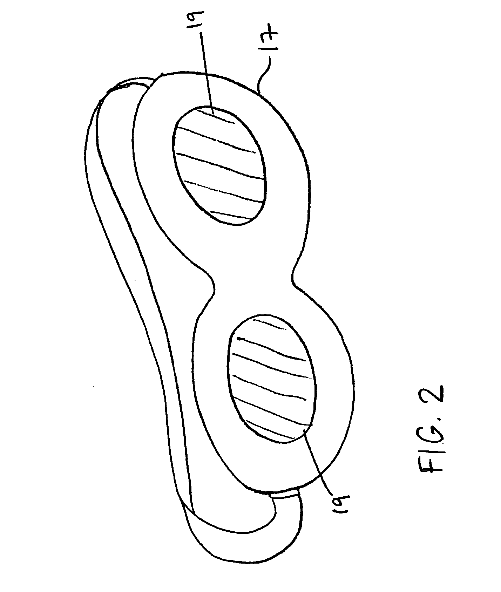

As shown in Figure 2, goggles 17 are provided to be worn by a user when viewing the flames 13. A sodium interference filter 19 is provided in each lens of the goggles as shown. The sodium interference filters have a pass band of approximately 1nm so that only light of wavelength 0.589µm passes through the filters. Thus, when a user wearing the goggles views the flames 13 of the burners 5, 9 through the window 15, only light from sodium trace elements in the burner flames is visible and the ambient light from the walls of the pyrolysis section which glow white hot is filtered out. Thus, the user can see the flames clearly and so can determine whether any adjustment to their size and/or orientation is required.

Any adjustments to the burner flames which are required are carried out by adjusting the quantity of fuel supplied to individual burners and/or the ratio of air to fuel supplied to the burners and/or by adjusting the direction of the jet of fuel emitted by the individual burners.

It will be appreciated that the embodiment of the invention described above is only a preferred embodiment thereof. Thus many variations could be made thereto without departing from the scope of the invention as claimed. For example, the interference filter could be provided in or on the window to the furnace itself or in a camera arranged inside the furnace rather then in the goggles described above.

It will therefore be appreciated that the above description is given by way of example only and is not intended to be limiting.

## Claims

1. A method of viewing the flame (13) produced by a burner (5,9) in a furnace, comprising viewing the flame (13) through an interference filter; wherein
the furnace is the pyrolysis section (1) of a petroleum cracker;
the fuel burnt by the burner (5,9) is natural gas; and
the interference filter is adapted to pass light of the wavelength of sodium only.

2. A method as claimed in claim 1, wherein the fuel burnt by the burner (5,9) is a mixture of hydrogen, methane and air.

3. A method as claimed in claim 1 or 2, wherein a window (15) is provided in the wall of the furnace through which the burner flame (13) can be viewed.

4. A method as claimed in claim 3, wherein the window (15) is made of quartz.

5. A method as claimed in claim 3 or 4, wherein the interference filter is provided as a panel attached to the window (15) of the furnace.

6. A method as claimed in claim 5, wherein the panel is hinged to the furnace so it can be placed over the window (15) or removed by a user as required.

7. A method as claimed in any preceding claim, wherein a pair of glasses or goggles having an interference filter in each lens thereof is provided.

8. A method as claimed in any preceding claim, wherein the interference filter is provided in a camera arranged inside the furnace and adapted to photograph the burner (5,9) at regular intervals.

9. A method as claimed in claim 8, wherein the information from the camera is relayed to an operator who makes any necessary adjustments to the burner (5,9) from a remote location.

10. A method as claimed in claim 8 or 9, wherein the camera is programmed to photograph the burner (5,9) about once every 10 minutes.

11. A method as claimed in any of claims 8 to 10, wherein the camera is programmed to move along a row of burners (5,9) and to photograph groups of one or more burner flames (13) in turn.

12. An apparatus comprising a furnace, a burner (5,9) in the furnace and an apparatus for viewing the flame (13) produced by the burner (5,9), the apparatus for viewing the flame (13) comprising an interference filter, wherein
the furnace is the pyrolysis section (1) of a petroleum cracker;
the burner (5,9) is for burning natural gas; and
the interference filter is adapted to pass light of the wavelength of sodium only.

13. An apparatus as claimed in claim 12, wherein a window (15) is provided in the wall of the furnace through which the burner flame (13) can be viewed.

14. An apparatus as claimed in claim 13, wherein the window (15) is made of quartz.

15. An apparatus as claimed in claim 13 or 14, wherein the interference filter is provided as a panel attached to the window (15) of the furnace.

16. An apparatus as claimed in claim 13, 14 or 15,
wherein the filter is a panel which can be placed over the window (15) or removed by a user as required.

17. An apparatus as claimed in any of claims 12 to 16, wherein the apparatus for viewing the flame (13) comprises a pair of glasses or goggles (17) comprising an interference filter (19) in each lens thereof.

18. An apparatus as claimed in any of claims 12 to 17, wherein the apparatus for viewing the flame (13) comprises a camera in which an interference filter is provided, wherein the camera is arranged inside the furnace and adapted to photograph the burner flame (13) at regular intervals.

19. An apparatus as claimed in claim 18, comprising means for relaying the information from the camera to an operator and means for making any necessary adjustments to the burner (5,9) from a remote location.

20. An apparatus as claimed in claim 18 or 19, wherein the camera is programmed to photograph the burner (5,9) about once every 10 minutes.

21. An apparatus as claimed in any of claims 18 to 20, wherein the camera is programmed to move along a row of burners (5,9) and to photograph groups of one or more burner flames (13) in turn.

## Patentansprüche

1. Verfahren zum Betrachten einer Flamme (13), die von einem Brenner (5,9) in einem Ofen erzeugt wird, das das Betrachten der Flamme (13) durch einen Interferenzfilter umfaßt, wobei
der Ofen der Pyrolyseabschnitt (1) eines Erdölcrackers ist;
der vom Brenner (5,9) verbrannte Brennstoff Erdgas ist; und
der Interferenzfilter so gestaltet ist, daß nur Licht mit der Wellenlänge von Natrium hindurchgeht.

2. Verfahren nach Anspruch 1, wobei der vom Brenner (5,9) verbrannte Brennstoff ein Gemisch von Wasserstoff, Methan und Luft ist.

3. Verfahren nach Anspruch 1 oder 2, wobei in der Wand des Ofens ein Fenster (15) vorgesehen ist, durch das die Brennerflamme (13) betrachtet werden kann.

4. Verfahren nach Anspruch 3, wobei das Fenster (15) aus Quarz hergestellt ist.

5. Verfahren nach Anspruch 3 oder 4, wobei der Interferenzfilter als Platte vorgesehen ist, die an das Fenster (15) des Ofens angebracht ist.

6. Verfahren nach Anspruch 5, wobei die Platte vom Anwender je nach Bedarf zum Ofen geschwenkt, so daß sie über dem Fenster (15) angeordnet werden kann, oder entfernt wird.

7. Verfahren nach einem der vorstehenden Ansprüche, wobei eine Brille oder Schutzbrille mit einem Interferenzfilter in jeder ihrer Linsen vorgesehen ist.

8. Verfahren nach einem der vorstehenden Ansprüche, wobei der Interferenzfilter in einer Kamera vorgesehen ist, die im Inneren des Ofens angeordnet ist und dazu dient, den Brenner (5,9) in regelmäßigen Abständen zu photographieren.

9. Verfahren nach Anspruch 8, wobei die Information von der Kamera für einen Betreiber verstärkt wird, der irgendwelche erforderlichen Regelungen des Brenners (5,9) von einer entfernten Stelle vornimmt.

10. Verfahren nach Anspruch 8 oder 9, wobei die Kamera so programmiert ist, daß sie den Brenner (5,9) etwa alle 10 Minuten photographiert.

11. Verfahren nach einem der Ansprüche 8 bis 10, wobei die Kamera so programmiert ist, daß sie sich entlang einer Reihe von Brennern (5,9) bewegt und Gruppen von einer oder mehreren Brennerflammen (13) der Reihe nach photographiert.

12. Vorrichtung, die einen Ofen, einen Brenner (5,9) in diesem Ofen und eine Vorrichtung zum Betrachten der Flamme (13), die vom Brenner (5,9) erzeugt wird, umfaßt, wobei die Vorrichtung zum Betrachten der Flamme (13) einen Interferenzfilter umfaßt, wobei
der Ofen der Pyrolyseabschnitt (1) eines Erdölcrackers ist;
der vom Brenner (5,9) verbrannte Brennstoff Erdgas ist; und
der Interferenzfilter so gestaltet ist, daß nur Licht mit der Wellenlänge von Natrium hindurchgeht.

13. Vorrichtung nach Anspruch 12, wobei in der Wand des Ofens ein Fenster (15) vorgesehen ist, durch das die Brennerflamme (13) betrachtet werden kann.

14. Vorrichtung nach Anspruch 13, wobei das Fenster (15) aus Quarz hergestellt ist.

15. Vorrichtung nach Anspruch 13 oder 14, wobei der Interferenzfilter als Platte vorgesehen ist, die an das Fenster (15) des Ofens angebracht ist.

16. Vorrichtung nach Anspruch 13, 14 oder 15, wobei der Filter eine Platte ist, die vom Anwender je nach Bedarf über dem Fenster (15) angeordnet oder davon entfernt werden kann.

17. Vorrichtung nach einem der Ansprüche 12 bis 16, wobei die Vorrichtung zum Betrachten der Flamme (13) eine Brille oder Schutzbrille (17) umfaßt, die einen Interferenzfilter (19) in jeder ihrer Linsen umfaßt.

18. Vorrichtung nach einem der Ansprüche 12 bis 17, wobei die Vorrichtung zum Betrachten der Flamme (13) eine Kamera umfaßt, in der ein Interferenzfilter vorgesehen ist, wobei die Kamera im Inneren des Ofens angeordnet ist und dazu dient, die Brennerflamme (13) in regelmäßigen Abständen so photographieren.

19. Vorrichtung nach Anspruch 18, die eine Einrichtung zum Verstärken der Information von der Kamera für einen Betreiber und eine Einrichtung zum Durchführen irgendwelcher erforderlichen Regelungen des Brenners (5,9) von einer entfernten Stelle aus umfaßt.

20. Vorrichtung nach Anspruch 18 oder 19, wobei die Kamera so programmiert ist, daß sie den Brenner (5,9) etwa alle 10 Minuten photographiert.

21. Vorrichtung nach einem der Ansprüche 18 bis 20, wobei die Kamera so programmiert ist, daß sie sich entlang einer Reihe von Brennern (5,9) bewegt und Gruppen von einer oder mehreren Brennerflammen (13) der Reihe nach photographiert.

## Revendications

1. Procédé permettant de visualiser la flamme (13) produite par un brûleur (5, 9) dans un four, comprenant la visualisation de la flamme (13) à travers un filtre d'interférence ;
dans lequel
le four est la section de pyrolyse (1) d'un craqueur de pétrole ;
le carburant brûlé par le brûleur (5, 9) est du gaz naturel ; et
le filtre d'interférence est adapté à laisser passer de la lumière de la longueur d'onde du sodium uniquement.

2. Procédé selon la revendication 1, dans lequel le carburant brûlé par le brûleur (5, 9) est un mélange d'hydrogène, de méthane et d'air.

3. Procédé selon la revendication 1 ou 2, dans lequel une fenêtre (15) est prévue dans la paroi du four à travers laquelle la flamme de brûleur (13) peut être visualisée.

4. Procédé selon la revendication 3, dans lequel la fenêtre (15) est composée de quartz.

5. Procédé selon la revendication 3 ou 4, dans lequel le filtre d'interférence est produit comme un panneau fixé à la fenêtre (15) du four.

6. Procédé selon la revendication 5, dans lequel le panneau est articulé au four de telle sorte qu'il peut être placé sur la fenêtre (15) ou retiré par un utilisateur si nécessaire.

7. Procédé selon l'une quelconque des revendications précédentes, dans lequel une paire de verres ou de lunettes comportant un filtre d'interférence dans chacune de ses lentilles est prévue.

8. Procédé selon l'une quelconque des revendications précédentes, dans lequel le filtre d'interférence est prévu dans un appareil photo agencé à l'intérieur du four et adapté pour photographier le brûleur (5, 9) à intervalles réguliers.

9. Procédé selon la revendication 8, dans lequel l'information depuis l'appareil photo est envoyée à un opérateur qui effectue les ajustements nécessaires quelconques au brûleur (5, 9) depuis un emplacement éloigné.

10. Procédé selon la revendication 8 ou 9, dans lequel l'appareil photo est programmé afin de photographier le brûleur (5, 9) environ une fois toutes les 10 minutes.

11. Procédé selon l'une quelconque des revendications 8 à 10, dans lequel l'appareil photo est programmé afin de se déplacer le long d'une rangée de brûleurs (5, 9) et afin de photographier des groupes d'une ou de plusieurs flammes de brûleur (13) tour à tour.

12. Appareil comprenant un four, un brûleur (5, 9) dans le four et un appareil destiné à visualiser la flamme (13) produite par le brûleur (5, 9), l'appareil destiné à visualiser la flamme (13) comprenant un filtre d'interférence, dans lequel
le four est la section de pyrolyse (1) d'un craqueur de pétrole ;
le brûleur (5, 9) est destiné à brûler du gaz naturel ; et
le filtre d'interférence est adapté à laisser passer de la lumière de la longueur d'onde du sodium uniquement.

13. Appareil selon la revendication 12, dans lequel une fenêtre (15) est prévue dans la paroi du four à travers lequel la flamme de brûleur (13) peut être visualisée.

14. Appareil selon la revendication 13, dans lequel la fenêtre (15) est composée de quartz.

15. Appareil selon la revendication 13 ou 14, dans lequel le filtre d'interférence est fourni comme un panneau fixé à la fenêtre (15) du four.

16. Appareil selon la revendication 13, 14 ou 15, dans lequel le filtre est un panneau qui peut être placé sur la fenêtre (15) ou retiré par un utilisateur si nécessaire.

17. Appareil selon l'une quelconque des revendications 12 à 16, dans lequel l'appareil destiné à visualiser la flamme (13) comprend une paire de verres ou de lunettes (17) comprenant un filtre d'interférence (19) dans chacune de ses lentilles.

18. Appareil selon l'une quelconque des revendications 12 à 17, dans lequel l'appareil destiné à visualiser la flamme (13) comprend un appareil photo dans lequel un filtre d'interférence est prévu, dans lequel l'appareil photo est agencé à l'intérieur du four et adapté à photographier la flamme du brûleur (13) à intervalles réguliers.

19. Appareil selon la revendication 18, comprenant des moyens destinés à fournir l'information depuis l'appareil photo vers un opérateur et des moyens destinés à faire les ajustements nécessaires quelconques au brûleur (5, 9) depuis un emplacement éloigné.

20. Appareil selon la revendication 18 ou 19, dans lequel l'appareil photo est programmé afin de photographier le brûleur (5, 9) environ une fois toutes les 10 minutes.

21. Appareil selon l'une quelconque des revendications 18 à 20, dans lequel l'appareil photo est programmé afin de se déplacer le long d'une rangée de brûleurs (5, 9) et afin de photographier des groupes d'une ou de plusieurs flammes de brûleur (13) tour à tour.
